# FASCICULE DE BREVET EUROPEEN

(11) **EP 0 574 312 B1**
(45) Date de publication et mention de la délivrance du brevet: **28.09.1994**
(21) Numéro de dépôt: 93401475.4
(22) Date de dépôt: 10.06.1993
(51) Int. Cl.: C07D 213/80, A61K 31/44

(54) **Nicotinates de diacylglycérols, leur procédé de préparation et les compositions pharmaceutiques qui les contiennent**
Diacylglycerol-Nicotinate, Verfahren zu ihrer Herstellung und diese enthaltende pharmazeutische Zubereitungen
Diacylglycerol nicotinates, processes for their preparation and pharmaceutical compositions containing them

(30) Priorité: 10.06.1992 FR 9206951
(43) Date de publication de la demande: 15.12.1993
(73) Titulaire: ADIR ET COMPAGNIE, 92415 Courbevoie Cédex (FR)
(72) Inventeur: Cordi, Alex, F-92150 Suresnes (FR); Lacoste, Jean-Michel, F-92310 Sevres (FR); Duhault, Jacques, F-78290 Croissy sur Seine (FR); Espinal, Joseph, F-75008 Paris (FR); Boulanger, Michelle, F-78400 Chatou (FR)

(56) Documents cités:
- FR-A- 2 476 095
- JOURNAL OF PHARMACOBIO-DYNAMICS. vol. 11, no. 8, 1988, TOKYO pages 555 - 562 J.SUGIHARA ET AL. 'Studies on intestinal lymphatic absorption of drugs. II. Glyceride prodrugs for improving lymphatic absorption of naproxen and nicotinic acid.'

## Description

La présente invention concerne de nouveaux nicotinates de diacylglycérol, leur procédé de préparation et les compositions pharmaceutiques qui les contiennent.

Certains nicotinates de diacylglycérol ont déjà été décrits, notamment par J. Sugihara et al. (J. Pharmacobio-Dyn., 11, 555-562, (1988)). Ces composés sont utilisés pour corriger l'élévation du taux de lipides plasmatiques, en particulier des triglycérides et du cholestérol des LDL. Toutefois, les composés décrits par J. Sugihara sont utilisés en doses importantes pour pallier la brièveté de leur action. Ceci entraîne des concentrations plasmatiques élevées responsables d'une vasodilatation périphérique provoquant "flush" et prurit. De plus, l'effet hypolipémiant, qui dure environ 4 heures, est suivi d'un rebond important.

Les nouveaux nicotinates de diacylglycérol décrits dans la présente demande ont été synthétisés dans le but d'éviter une absorption rapide conduisant à l'effet "flush" et d'obtenir des concentrations plasmatiques plus faibles et plus durables évitant le phénomène de rebond.

Plus spécifiquement, la présente invention concerne de nouveaux nicotinates de diacylglycérol de formule (I) : 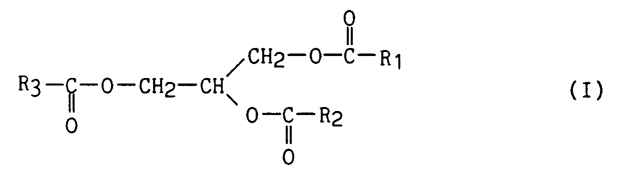
dans laquelle :
- R₁ et R₂: identiques ou différents représentent un radical alkyle (C₁₁-C₁₉) linéaire ou ramifié ou alkényle (C₁₁-C₁₉) linéaire ou ramifié,
- R₃: représente un radical pyridin-3-yl, 2-méthyl-pyrazin-5-yl ou 2-méthyl-1(N-oxyde)pyrazin-5-yl,
leurs énantiomères et éventuels diastéréoisomères ainsi que leurs sels d'addition à un acide pharmaceutiquement acceptable.

Parmi les acides pharmaceutiquement acceptables on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

L'invention s'étend également au procédé de préparation des composés de formule (I) caractérisé en ce que l'on fait réagir sur le 1,3-dioxolane de formule (II) sous forme racémique ou d'énantiomère pur : 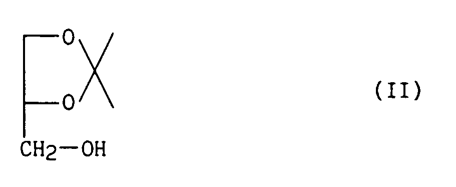
le chlorure d'acide de formule (III) :

R'₃ - CO - Cl (III)

dans laquelle R'₃ représente un radical pyridin-3-yl ou 2-méthyl-pyrazin-5-yl,
pour conduire au 1,3-dioxolane de formule (IV) : 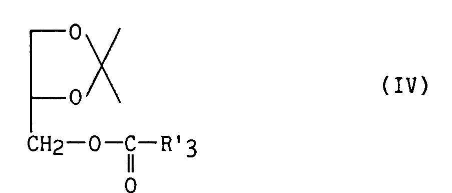
dans laquelle R'₃ a la même signification que précédemment,
que l'on transforme en diol correspondant de formule (V), en milieu acide : 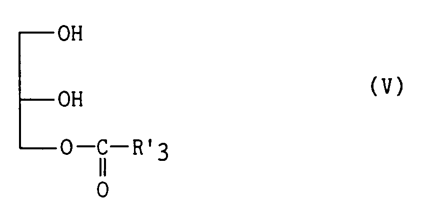
dans laquelle R'₃ a la même signification que précédemment,
que l'on fait réagir selon la nature des composés de formule (I) que l'on souhaite obtenir avec :
- soit dans le cas où R₁ et R₂ sont identiques :
   2 équivalents du chlorure d'acide de formule (VI), en présence d'une base,

   R₁ - CO - Cl (VI)

   dans laquelle R₁ a la même signification que dans la formule (I), pour conduire au composé de formule (I/a), cas particulier des composés de formule (I), 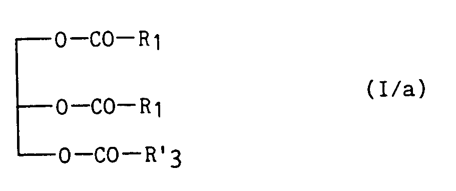 dans laquelle R₁ et R'₃ ont la même signification que précédemment,
- soit dans le cas où R₁ et R₂ sont différents :
   avec un équivalent du chlorure d'acide de formule (VI) décrit précédemment,
   pour conduire, après séparation des composés mono et diacylés, au composé de formule : 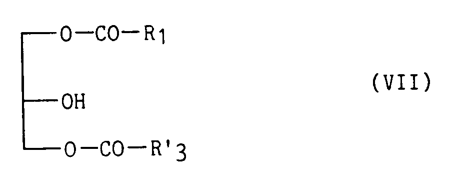 dans laquelle R₁ et R'₃ ont la même signification que précédemment,
   que l'on fait réagir avec un équivalent du chlorure d'acide de formule (VIII) en présence d'une base :

   R₂ - CO - Cl (VIII)

   pour conduire au compposé de formule (I/b), cas particulier des composés de formule (I), 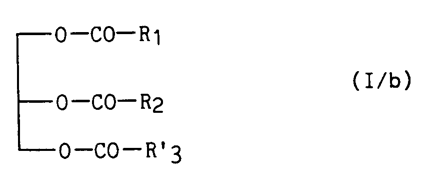 dans laquelle R₁, R₂ et R'₃ ont la même signification que précédemment,
composés de formule (I/a) ou (I/b), qui lorsque R'₃ représente un radical 2-méthyl-pyrazin-5-yl peuvent subir une oxydation par de l'eau oxygénée en milieu acétique, pour conduire respectivement aux composés de formule (I/c) et (I/d), cas particuliers des composés de formule (I) : 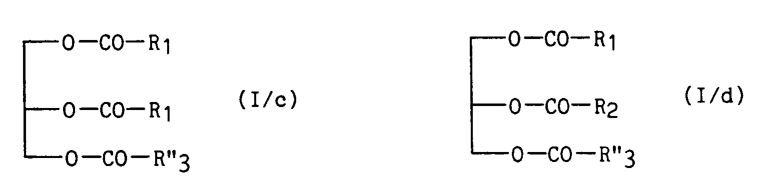
dans lesquelles R₁ et R₂ ont la même signification que dans la formule (I) et R''₃ représente un radical 2-méthyl-1-(N-oxyde)pyrazin-5-yl,
composés de formule (I/a), (I/b), (I/c) ou (I/d) :
- que l'on purifie, le cas échéant, selon une technique classique de purification,
- dont on sépare, si on le souhaite, les isomères selon une technique classique de purification,
- et que l'on transforme, éventuellement, en leurs sels d'addition à un acide pharmaceutiquement acceptable.

Les composés de formule (I) dans laquelle R₁=R₂ peuvent également être obtenus selon le procédé caractérisé en ce que l'on fait réagir un composé de formule (IX), sous forme racémique ou d'énantiomère pur (obtenu selon le procédé décrit par F.R. PFEIFFER et coll., Tet. Lett., 32, 3549-3552, 1968) : 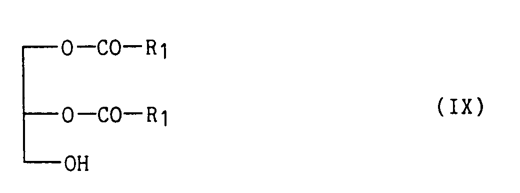
dans laquelle R₁ a la même signification que dans la formule (I), sur le chlorure d'acide de formule (III), en présence d'une base :

R'₃ - CO - Cl (III)

dans laquelle R'₃ représente un radical pyridin-3-yl ou 2-méthylpyrazin-5-yl, pour conduire au composé de formule (I/a), cas particulier des composés de formule (I) : 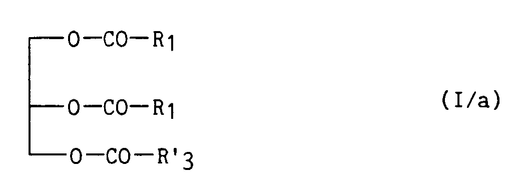
dans laquelle R₁ et R'₃ ont la même signification que précédemment,
composé de formule (I/a), qui lorsque R'₃ représente un radical 2-méthylpyrazin-5-yl peut subir une oxydation par de l'eau oxygénée en milieu acétique, pour conduire au composé de formule (I/c), cas particulier des composés de formule (I) : 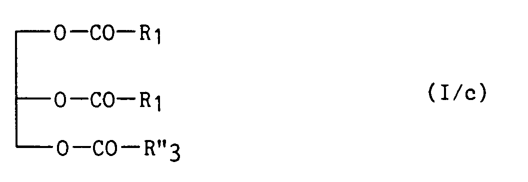
dans laquelle R₁ a la même signification que dans la formule (I) et R''₃ représente un radical 2-méthyl-1-(N-oxyde)pyrazin-5-yl,
composés de formule (I/a) ou (I/c) :
- que l'on purifie, le cas échéant, selon une technique classique de purification,
- dont on sépare, si on le souhaite, les isomères selon une technique classique de purification,
- et que l'on transforme, éventuellement, en leurs sels d'addition à un acide pharmaceutiquement acceptable.

Ces nouveaux nicotinates de diacylglycérol présentent des propriétés pharmacologiques très intéressantes. En particulier, ils diminuent la concentration en lipides plasmatiques et, de ce fait, sont utilisables dans la prévention de l'athérosclérose.

Par rapport aux nicotinates connus, ces nouveaux produits évitent le pic précoce de concentration initiale d'acide nicotinique et n'entraînent pas d'effet rebond.

L'invention s'étend aussi aux compositions pharmaceutiques renfermant comme principe actif au moins un composé de formule (I) ou ses isomères optiques avec un ou plusieurs excipients inertes, non toxiques et appropriés. Les compositions pharmaceutiques ainsi obtenues pourront être présentées sous diverses formes, les plus avantageuses étant les comprimés, les dragées, les gélules, suppositoires, suspensions buvables, les formes transdermiques (gel, patch), etc...

La posologie utile est adaptable selon la nature et la sévérité de l'affection, la voie d'administration ainsi que selon l'âge et le poids du patient. Cette posologie unitaire varie de 0,5 g à 5 g par jour en une ou plusieurs prises.

Les exemples suivants illustrent l'invention et ne la limitent en aucune façon.

Les produits de départ utilisés sont des produits connus ou préparés selon des modes opératoires connus.

### Exemple 1 : Glycérol-1,2-distéarate-3-nicotinate

### Stade A : Glycérol-1,2-isopropylidène-3-nicotinate

A une solution maintenue à 0°C, sous agitation, contenant 80 mmoles de 1,2-isopropylidène glycérol dans 100 ml de chloroforme sont additionnées, goutte à goutte, 160 mmoles de triéthylamine puis 80 mmoles de chlorhydrate du chlorure de nicotinoyle. Après 12 heures d'agitation à 20°C, le mélange est dilué par 300 ml d'éther éthylique puis lavé par 100 ml d'eau, 100 ml d'une solution aqueuse à 5 % de bicarbonate de sodium et à nouveau par 100 ml d'eau. La phase organique est séchée et concentrée sous vide.
Le produit attendu est obtenu, sous forme d'huile, après purification du résidu par chromatographie sur gel de silice en utilisant le dichlorométhane comme éluant.
Point d'ébullition : 148-150°C (p = 26,66 Pa)

### Stade B : Glycérol-3-nicotinate

Une suspension contenant 21 mmoles du produit obtenu au stade précédent dans 30 ml d'acide acétique aqueux à 10 % est chauffée 3 heures à 100°C.

Après refroidissement et concentration, le résidu huileux obtenu est purifié par chromatographie sur gel de silice en utilisant comme éluant un mélange dichlorométhane/méthanol (93/7) et conduit au produit attendu sous forme d'un solide blanc.
Point de fusion : 87-88°C

### Stade C : Glycérol-1,2-distéarate-3-nicotinate

Une solution contenant 24 mmoles de chlorure de stéaroyle dans 30 ml de chloroforme est additionnée goutte à goutte et sous agitation à une solution refroidie à 0°C contenant 12 mmoles du produit obtenu au stade précédent et 24 mmoles de pyridine dans 30 ml de chloroforme. Le mélange réactionnel est agité à 20°C pendant une nuit puis lavé par de l'eau, séché et évaporé sous vide. Le résidu huileux obtenu est purifié par chromatographie sur gel de silice en utilisant comme éluant un mélange dichlorométhane/acétone (98/2) et conduit au produit attendu, sous forme d'un solide blanc après recristallisation dans l'hexane.
Point de fusion : 67-69°C

| Microanalyse élémentaire : | | | |
|---|---|---|---|
| | C % | H % | N % |
| calculé | 74,03 | 10,91 | 1,92 |
| trouvé | 74,06 | 10,72 | 2,07 |

Les exemples suivants ont été obtenus en utilisant le même mode opératoire que celui décrit pour l'exemple 1.

### Exemple 2 : Glycérol-1,2-dimyristate-3-nicotinate

Point de fusion : 49-51°C (hexane)

| Microanalyse élémentaire : | | | |
|---|---|---|---|
| | C % | H % | N % |
| calculé | 71,92 | 10,28 | 2,27 |
| trouvé | 71,99 | 10,49 | 2,38 |

### Exemple 3 : Glycérol-1,2-dipalmitate-3-nicotinate

Point de fusion : 64-66°C (hexane)

| Microanalyse élémentaire : | | | |
|---|---|---|---|
| | C % | H % | N % |
| calculé | 73,06 | 10,62 | 2,08 |
| trouvé | 72,99 | 10,54 | 2,13 |

### Exemple 4 : Glycérol-(d)-1,2-dipalmitate-3-nicotinate

A une solution maintenue à 0°C, contenant 5,27 mmoles de (d)-1,2-dipalmitoylglycérol (préparé selon le procédé décrit dans Tet. Lett., 32, 3549-3559, 1968) dans 50 ml de chloroforme sont additionnées successivement 15,8 mmoles de triéthylamine, 0,4 mmoles de diméthylaminopyridine et 7,9 mmoles du chlorhydrate de chlorure de nicotinoyle. Après 12 heures d'agitation à 20°C, le mélange réactionnel est lavé par de l'eau, séché et concentré. Le résidu obtenu est purifié par chromatographie sur colonne de silice en utilisant comme éluant un mélange dichlorométhane/acétone (98/2) et conduit au produit attendu qui est recristallisé dans l'hexane.
Point de fusion : 64-66°C
Pouvoir rotatoire : [α]_{D}²⁰ = + 1,2° (C = 1 %, CHCl₃)

| Microanalyse élémentaire : | | | |
|---|---|---|---|
| | C % | H % | N % |
| calculé | 73,06 | 10,62 | 2,08 |
| trouvé | 73,31 | 10,26 | 2,18 |

### Exemple 5 : Glycérol-(l)-1,2-dipalmitate-3-nicotinate

Ce composé a été obtenu en utilisant le même procédé que celui décrit dans l'exemple 4 à partir de (l)-1,2-dipalmitoylglycérol.
Point de fusion : 64-66°C
Pouvoir rotatoire : [α]_{D}²⁰ = - 1,2° (C = 1 %, CHCl₃)

| Microanalyse élémentaire : | | | |
|---|---|---|---|
| | C % | H % | N % |
| calculé | 73,06 | 10,62 | 2,08 |
| trouvé | 73,29 | 10,70 | 2,22 |

### Etude pharmacologique des dérivés de l'invention

### Exemple 6 : Etude de la diminution de la concentration en lipides plasmatiques

a Cette étude a été réalisée sur des rats SDCD mâles âgés de dix semaines, pesant de 325 à 350 g, acclimatés pendant une dizaine de jours et mis à jeun la veille de l'expérience. L'administration des produits est effectuée par gavage avec sonde oesophagienne, chaque animal recevant 2 ml/kg d'une suspension de 0,4 mM/kg de produit actif dans la Gomme du Sénégal (à 20 % dans l'eau distillée).
   30 minutes, 1 heure, 2 heures, 4 heures, 6 heures et 8 heures après administration, les animaux sont sacrifiés par décapitation. Les paramètres mesurés sont les taux des triglycérides et des acides gras libres ainsi que la concentration sanguine de l'acide nicotinique.
   Les courbes 1 et 2 (en annexe) montrent l'efficacité des produits de l'invention à l'égard des triglycérides et de la concentration sanguine en acide nicotinique. La courbe 3 (en annexe) montre l'effet des produits de l'invention à l'égard des acides gras libres et met en évidence l'absence d'effet rebond, notamment pour le composé de l'exemple 1.
b Les résultats rassemblés dans le tableau ci-dessous montrent les concentrations en triglycérides et en acides gras libres obtenues après administration, à la dose de 0,1 mmole/kg P.O., du composé de l'exemple 3 et de ses deux énantiomères (exemples 4 et 5). L'étude a été réalisée sur des lots de 12 animaux.

| Taux de triglycérides (mg/l) | | | | | |
|---|---|---|---|---|---|
| Temps (heures) | 1 | 2 | 4 | 6 | 8 |
| Témoins Gomme du Sénégal | 539±49 | 598±49 | 652±47 | 508±32 | 495±49 |
| Acide nicotinique | 190±19 | 498±60 | 568±25 | 533±40 | 623±51 |
| Exemple 3 | 192±22 | 263±30 | 462±43 | 466±33 | 503±28 |
| Exemple 4 | 183±22 | 238±29 | 478±58 | 528±58 | 499±25 |
| Exemple 5 | 196±24 | 279±31 | 398±48 | 545±33 | 544±18 |

| Taux des acides gras libres (mmole/l) | | | | | |
|---|---|---|---|---|---|
| Temps (heures) | 1 | 2 | 4 | 6 | 8 |
| Témoins Gomme du Sénégal | 0,727±0,031 | 0,803±0,054 | 0,833±0,061 | 0,859±0,050 | 0,948±0,052 |
| Acide nicotinique | 0,101±0,010 | 1,207±0,100 | 1,068±0,069 | 0,974±0,078 | 1,026±0,094 |
| Exemple 3 | 0,111±0,014 | 0,265±0,095 | 1,068±0,059 | 1,117±0,068 | 1,194±0,076 |
| Exemple 4 | 0,133±0,026 | 0,257±0,052 | 1,118±0,103 | 1,163±0,062 | 1,040±0,077 |
| Exemple 5 | 0,105±0,026 | 0,363±0,082 | 1,051±0,072 | 1,240±0,085 | 1,130±0,055 |

### Exemple 7 : Composition pharmaceutique

Formule de préparation pour 1000 comprimés dosés à 0,5 g

| | |
|---|---|
| Glycérol-1,2-distéarate-3-nicotinate | 500 g |
| Hydroxypropylcellulose | 2 g |
| Amidon de blé | 10 g |
| Lactose | 100 g |
| Stéarate de magnésium | 3 g |
| Talc | 3 g |

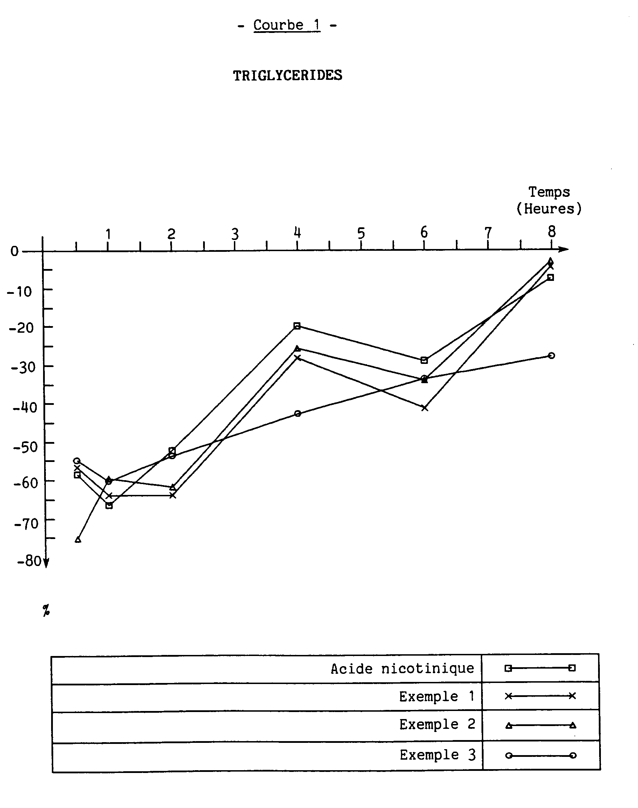

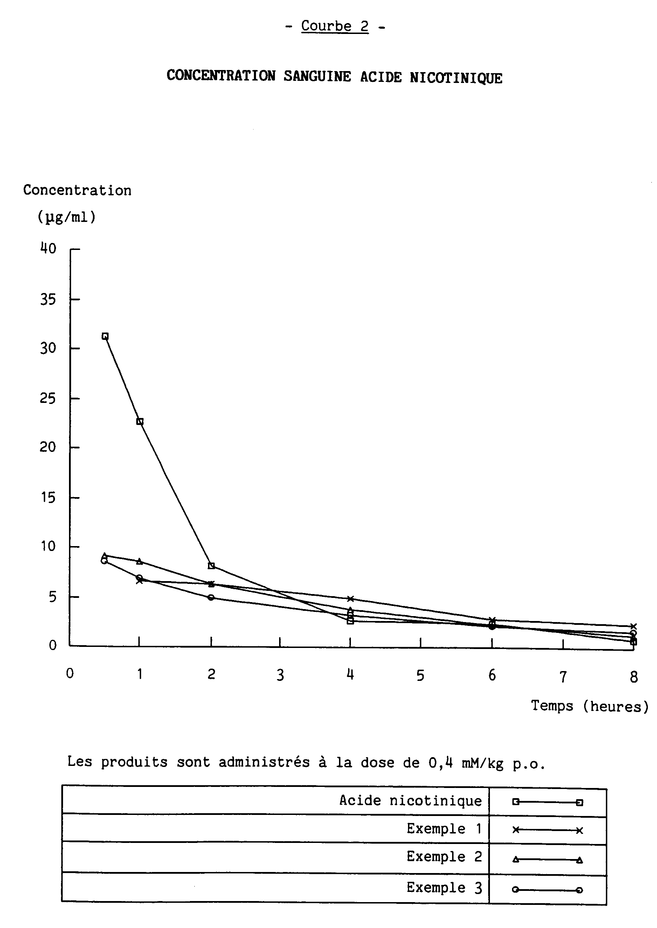

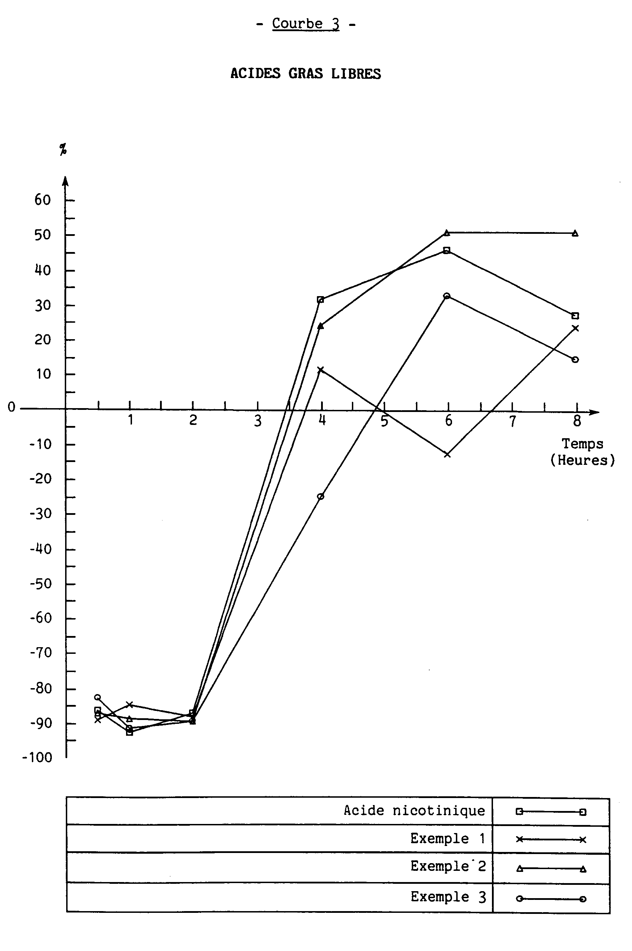

## Revendications

1. Composés de formule (I) : 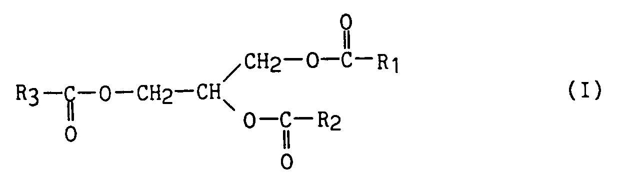 dans laquelle :
R₁ et R₂ identiques ou différents représentent un radical alkyle (C₁₁-C₁₉) linéaire ou ramifié ou alkényle (C₁₁-C₁₉) linéaire ou ramifié,
R₃ représente un radical pyridin-3-yl, 2-méthyl-pyrazin-5-yl ou 2-méthyl-1-(N-oxyde)pyrazin-5-yl,
leurs énantiomères et éventuels diastéréoisomères ainsi que leurs sels d'addition à un acide pharmaceutiquement acceptable.

2. Composés de formule (I) selon la revendication 1 tels que R₁ et R₂ sont identiques.

3. Composés de formule (I) selon l'une quelconque des revendications 1 et 2 tels que R₁ et R₂ représentent un radical alkyle (C₁₁-C₁₉) linéaire ou ramifié.

4. Composés de formule (I) selon la revendication 1 tels que R₃ représente un radical pyridin-3-yl.

5. Composé de formule (I) selon la revendication 1 qui est le glycérol-1,2-dipalmitate-3-nicotinate et ses énantiomères.

6. Procédé de préparation des composés de formule (I) selon la revendication 1 caractérisé en ce que l'on fait réagir sur le 1,3-dioxolane de formule (II) : 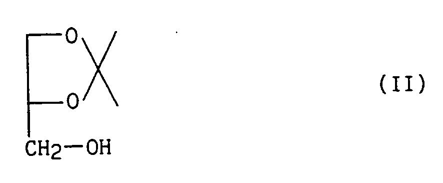 le chlorure d'acide de formule (III) :
R'₃ - CO - Cl (III)
dans laquelle R'₃ représente un radical pyridin-3-yl ou 2-méthyl-pyrazin-5-yl,
pour conduire au 1,3-dioxolane de formule (IV) : 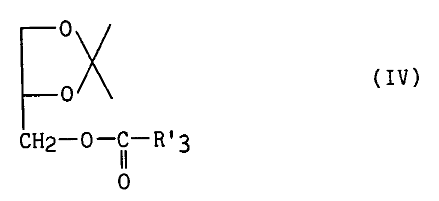 dans laquelle R'₃ a la même signification que précédemment,
que l'on transforme en diol correspondant de formule (V), en milieu acide : 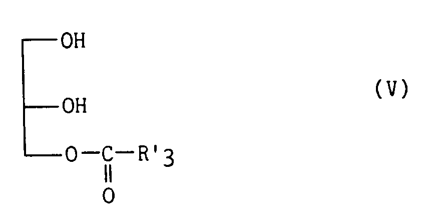 dans laquelle R'₃ a la même signification que précédemment,
que l'on fait réagir selon la nature des composés de formule (I) que l'on souhaite obtenir avec :
- soit dans le cas où R₁ et R₂ sont identiques :
2 équivalents du chlorure d'acide de formule (VI), en présence d'une base,
R₁ - CO - Cl (VI)
dans laquelle R₁ a la même signification que dans la formule (I),
pour conduire au composé de formule (I/a), cas particulier des composés de formule (I), 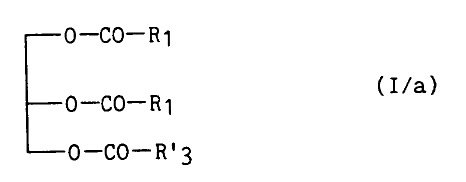 dans laquelle R₁ et R'₃ ont la même signification que précédemment,
- soit dans le cas où R₁ et R₂ sont différents :
avec un équivalent du chlorure d'acide de formule (VI) décrit précédemment,
pour conduire, après séparation des composés mono et diacylés, au composé de formule : 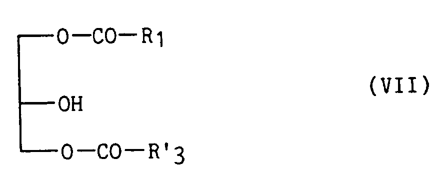 dans laquelle R₁ et R'₃ ont la même signification que précédemment,
que l'on fait réagir avec un équivalent du chlorure d'acide de formule (VIII) en présence d'une base :
R₂ - CO - Cl (VIII)
pour conduire au compposé de formule (I/b), cas particulier des composés de formule (I), 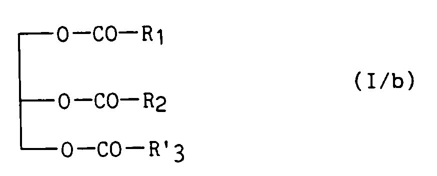 dans laquelle R₁, R₂ et R'₃ ont la même signification que précédemment,
composés de formule (I/a) ou (I/b), qui lorsque R'₃ représente un radical 2-méthyl-pyrazin-5-yl peuvent subir une oxydation par de l'eau oxygénée en milieu acétique, pour conduire respectivement aux composés de formule (I/c) et (I/d), cas particulier des composés de formule (I) : 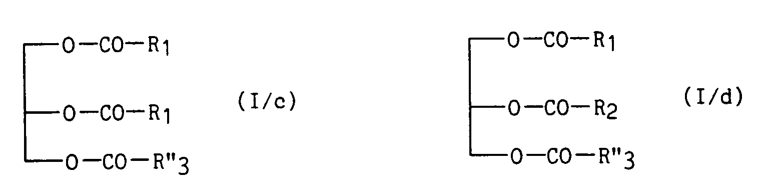 dans lesquelles R₁ et R₂ ont la même signification que dans la formule (I) et R''₃ représente un radical 2-méthyl-1-(N-oxyde)pyrazin-5-yl,
composés de formule (I/a), (I/b), (I/c) et (I/d) :
- que l'on purifie, le cas échéant, selon une technique classique de purification,
- dont on sépare, si on le souhaite, les isomères selon une technique classique de purification,
- et que l'on transforme, éventuellement, en leurs sels d'addition à un acide pharmaceutiquement acceptable.

7. Procédé de préparation des composés de formule (I) selon la revendication 1 dans laquelle R₁=R₂ caractérisé en ce que l'on fait réagir un composé de formule (IX), sous forme racémique ou d'énantiomère pur : 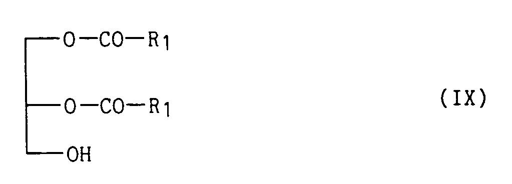 dans laquelle R₁ a la même signification que dans la formule (I),
sur le chlorure d'acide de formule (III), en présence d'une base :
R'₃ - CO - Cl (III)
dans laquelle R'₃ représente un radical pyridin-3-yl ou 2-méthylpyrazin-5-yl, pour conduire au composé de formule (I/a), cas particulier des composés de formule (I) : 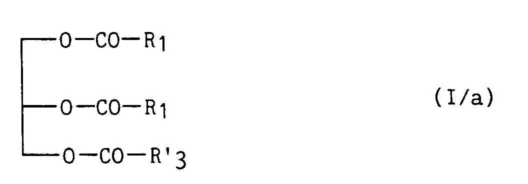 dans laquelle R₁ et R'₃ ont la même signification que précédemment,
composé de formule (I/a), qui lorsque R'₃ représente un radical 2-méthylpyrazin-5-yl peut subir une oxydation par de l'eau oxygénée en milieu acétique, pour conduire au composé de formule (I/c), cas particulier des composés de formule (I) : 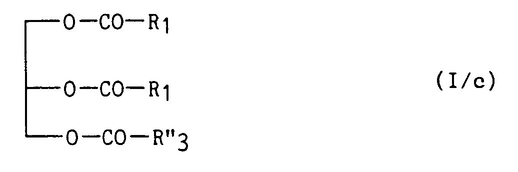 dans laquelle R₁ a la même signification que dans la formule (I) et R''₃ représente un radical 2-méthyl-1-(N-oxyde)pyrazin-5-yl,
composés de formule (I/a) ou (I/c) :
- que l'on purifie, le cas échéant, selon une technique classique de purification,
- dont on sépare, si on le souhaite, les isomères selon une technique classique de purification,
- et que l'on transforme, éventuellement, en leurs sels d'addition à un acide pharmaceutiquement acceptable.

8. Compositions pharmaceutiques contenant comme principe actif au moins un composé selon l'une des quelconques revendications 1 à 5, seul ou en combinaison avec un ou plusieurs véhicules inertes, non toxiques pharmaceutiquement acceptables.

9. Compositions pharmaceutiques selon la revendication 8 utiles dans le traitement préventif de l'athérosclérose.

## Claims

1. Compounds of formula (I): 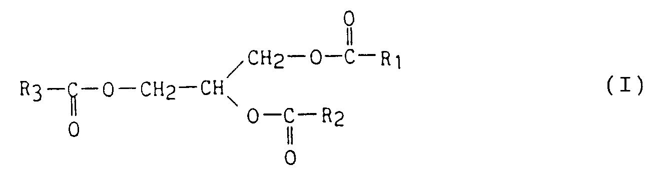 in which:
R₁ and R₂, which may be the same or different, represent a linear or branched (C₁₁-C₁₉)-alkyl radical or a linear or branched (C₁₁-C₁₉)-alkenyl radical, and
R₃ represents a pyridin-3-yl radical, a 2-methylpyrazin-5-yl radical or a 2-methyl-1-(N-oxide)pyrazin-5-yl radical,
their enantiomers and, where appropriate, their diastereoisomers, as well as their addition salts with a pharmaceutically acceptable acid.

2. Compounds of formula (I) according to claim 1 in which R₁ and R₂ are the same.

3. Compounds of formula (I) according to either claim 1 or claim 2 in which R₁ and R₂ represent a linear or branched (C₁₁-C₁₉)-alkyl radical.

4. Compounds of formula (I) according to claim 1 in which R₃ represents a pyridin-3-yl radical.

5. A compound of formula (I) according to claim 1 which is glycerol 1,2-dipalmitate 3-nicotinate, and its enantiomers.

6. Process for the preparation of the compounds of formula (I) according to claim 1, characterised in that there is reacted with the 1,3-dioxolane of formula (II): 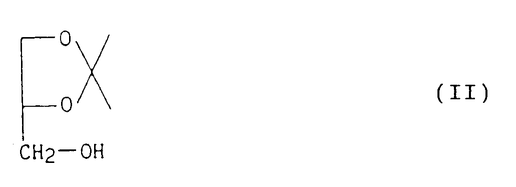 the acid chloride of formula (III):
R'₃-CO-Cl (III),
in which R'₃ represents a pyridin-3-yl radical or a 2-methylpyrazin-5-yl radical,
to give the 1,3-dioxolane of formula (IV): 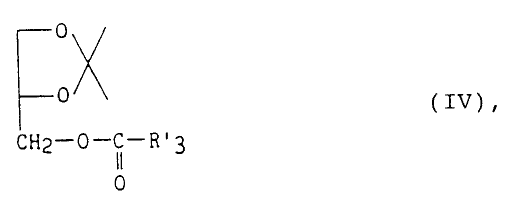 in which R'₃ has the same meaning as above,
which is converted, in an acidic medium, into the corresponding diol of formula (V): 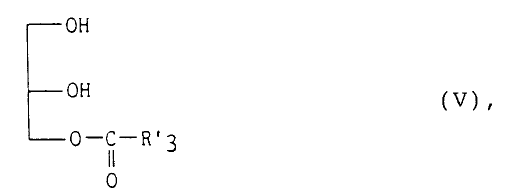 in which R'₃ has the same meaning as above,
which is reacted, depending upon the nature of the compounds of formula (I) which are to be obtained:
- either, in the case where R₁ and R₂ are the same: with 2 equivalents of the acid chloride of formula (VI), in the presence of a base,
R₁-CO-Cl (VI),
in which R₁ has the same meaning as in formula (I),
to give the compound of formula (I/a), which is a particular case of the compounds of formula (I): 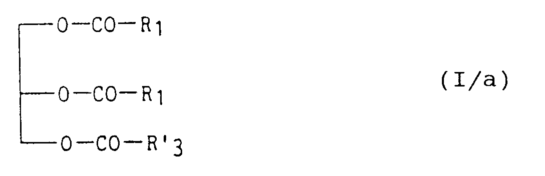 in which R₁ and R'₃ have the same meaning as above,
- or, in the case where R₁ and R₂ are different:
with one equivalent of the acid chloride of formula (VI) described above,
to give, after separation of the mono- and di-acylated compounds, the compound of the formula: 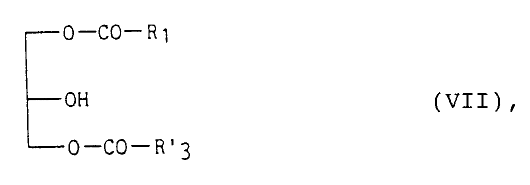 in which R₁ and R'₃ have the same meaning as above,
which is reacted, in the presence of a base, with one equivalent of the acid chloride of formula (VIII):
R₂-CO-Cl (VIII)
to give the compound of formula (I/b), which is a particular case of the compounds of formula (I): 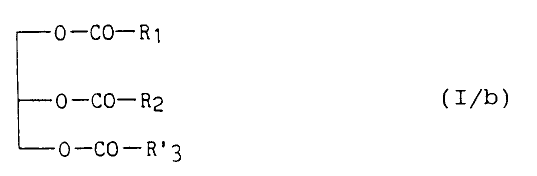 in which R₁, R₂ and R'₃ have the same meaning as above,
which compounds of formula (I/a) or (I/b), when R'₃ represents a 2-methylpyrazin-5-yl radical, may be subjected to oxidation by means of hydrogen peroxide in an acetic medium to give the compounds of formulae (I/c) and (I/d), respectively, which are a particular case of the compounds of formula (I): 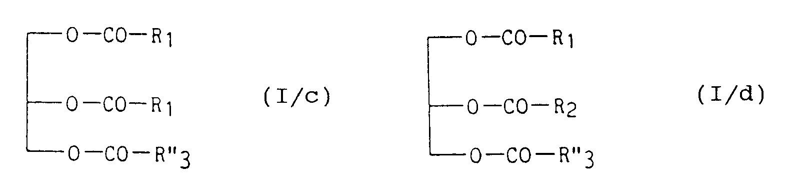 in which R₁ and R₂ have the same meaning as in formula (I) and R''₃ represents a 2-methyl-1-(N-oxide)pyrazin-5-yl radical,
which compounds of formulae (I/a), (I/b), (I/c) and (I/d):
- are purified, where applicable, by a conventional purification method,
- the isomers of which are separated, if desired, by a conventional purification method,
- and which are optionally converted into their addition salts with a pharmaceutically acceptable acid.

7. Process for the preparation of the compounds of formula (I) according to claim 1 in which R₁ = R₂, characterised in that a compound of formula (IX), in racemic form or in the form of a pure enantiomer, 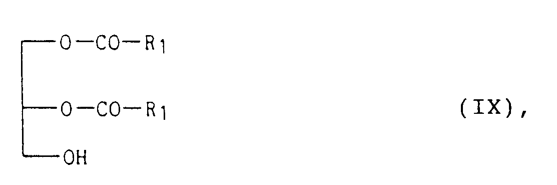 in which R₁ has the same meaning as in formula (I),
is reacted, in the presence of a base, with the acid chloride of formula (III):
R'₃-CO-Cl (III),
in which R'₃ represents a pyridin-3-yl radical or a 2-methylpyrazin-5-yl radical,
to give the compound of formula (I/a), which is a particular case of the compounds of formula (I): 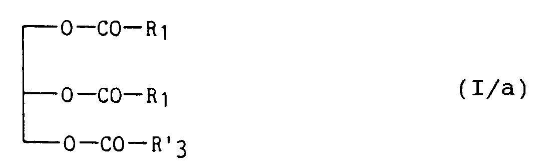 in which R₁ and R'₃ have the same meaning as above, which compound of formula (I/a), when R'₃ represents a 2-methylpyrazin-5-yl radical, may be subjected to oxidation by means of hydrogen peroxide in an acetic medium to give the compound of formula (I/c), which is a particular case of the compounds of formula (I): 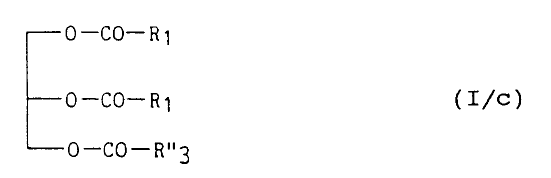 in which R₁ has the same meaning as in formula (I) and R''₃ represents a 2-methyl-1-(N-oxide)pyrazin-5-yl radical,
which compounds of formulae (I/a) or (I/c):
- are purified, where applicable, by a conventional purification method,
- the isomers of which are separated, if desired, by a conventional purification method,
- and which are optionally converted into their addition salts with a pharmaceutically acceptable acid.

8. Pharmaceutical compositions containing as active ingredient at least one compound according to any one of claims 1 to 5, on its own or in combination with one or more inert, non-toxic, pharmaceutically acceptable carriers.

9. Pharmaceutical compositions according to claim 8 for use in the preventive treatment of atherosclerosis.

## Patentansprüche

1. Verbindungen der Formel (I): 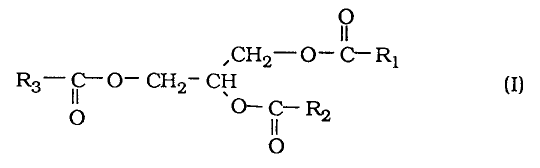 in der:
R₁ und R₂, die gleichartig oder verschieden sind, jeweils geradkettige oder verzweigte (C₁₁-C₁₉)-Alkylgruppen oder geradkettige oder verzweigte (C₁₁-C₁₉)-Alkenylgruppen und
R₃ eine Pyridin-3-yl-gruppe, eine 2-Methyl-pyrazin-5-yl-gruppe oder eine 2-Methyl-1-(N-oxid)-pyrazin-5-yl-gruppe bedeuten,
deren Enantiomere und möglichen Diastereoisomere sowie deren Additionssalze mit einer pharmazeutisch annehmbaren Säure.

2. Verbindungen der Formel (I) nach Anspruch 1, worin R₁ und R₂ gleich sind.

3. Verbindungen der Formel (I) nach einem der Ansprüche 1 und 2, worin R₁ und R₂ eine geradkettige oder verzweigte (C₁₁-C₁₉)-Alkylgruppe bedeuten.

4. Verbindungen der Formel (I) nach Anspruch 1, worin R₃ eine Pyridin-3-yl-gruppe bedeutet.

5. Verbindung der Formel (I) nach Anspruch 1, nämlich Glycerin-1,2-dipalmitat-3-nicotinat und dessen Enantiomere.

6. Verfahren zur Herstellung der Verbindungen der Formel (I) nach Anspruch 1, **dadurch gekennzeichnet,** daß man 1,3-Dioxolan der Formel (II): 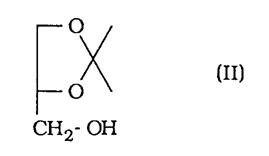 mit dem Säurechlorid der Formel (III):
R'₃ - CO - Cl (III)
in der R'₃ eine Pyridin-3-yl-gruppe oder eine 2-Methyl-pyrazin-5-yl-gruppe darstellt, umsetzt
zur Bildung des 1,3-Dioxolans der Formel (IV): 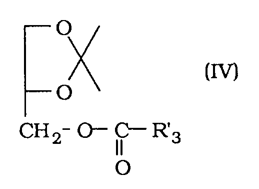 in der R'₃ die oben angegebenen Bedeutungen besitzt,
welches man in saurem Medium in das entsprechende Diol der Formel (V): 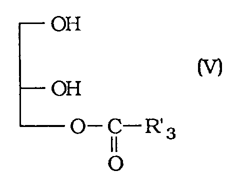 in der R'₃ die oben angegebenen Bedeutungen besitzt, umwandelt,
welches man in Abhängigkeit von der Art der herzustellenden Verbindungen der Formel (I):
- entweder, wenn R₁ und R₂ identisch sind:
mit 2 Äquivalenten des Säurechlorids der Formel (VI)
R₁ - CO - Cl (VI)
in der R₁ die bezüglich der Formel (I) angegebenen Bedeutungen besitzt, in Gegenwart einer Base umsetzt zur Bildung der Verbindung der Formel (I/a), einem Sonderfall der Verbindungen der Formel (I): 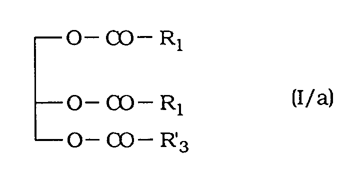 In der R₁ und R'₃ die oben angegebenen Bedeutungen besitzen.
- oder, wenn R₁ und R₂ verschieden sind:
mit einem Äquivalent des oben beschriebenen Säurechlorids der Formel (VI) umsetzt, so daß man nach der Trennung der mono- und diacylierten Verbindungen die Verbindung der Formel: 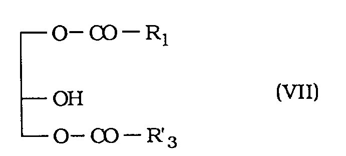 in der R₁ und R'₃ die oben angegebenen Bedeutungen besitzen, erhält, welche man mit einem Äquivalent des Säurechlorids der Formel (VIII):
R₂ - CO - Cl (VIII)
in Gegenwart einer Base umsetzt zur Bildung der Verbindung der Formel (I/b), einem Sonderfall der Verbindungen der Formel (I): 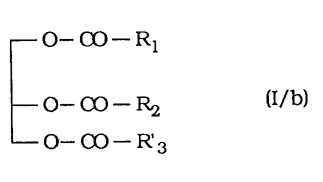 in der R₁, R₂ und R'₃ die oben angegebenen Bedeutungen besitzen,
welche Verbindung der Formel (I/a) oder (I/b), wenn R'₃ eine 2-Methyl-pyrazin-5-yl-gruppe darstellt, einer Oxidation mit Wasserstoffperoxid in essigsaurem Medium unterworfen werden kann zur Bildung der Verbindungen der Formel (I/c) bzw. (I/d), einem Sonderfall der Verbindungen der Formel (I): 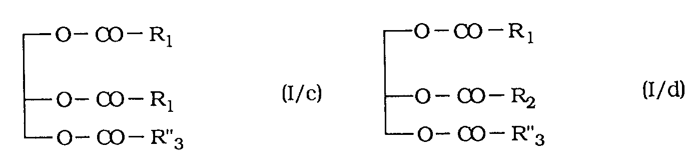 worin R₁ und R₂ die bezüglich der Formel (I) angegebenen Bedeutungen besitzen und R''₃ eine 2-Methyl-1-(N-oxid)-pyrazin-5-yl-gruppe darstellt,
welche Verbindungen der Formeln (I/a), (I/b), (I/c) und (I/d):
- man gegebenenfalls nach einer klassichen Reinigungsmethode reinigt,
- gewünschtenfalls mit Hilfe einer klassischen Reinigungsmethode in die Isomeren auftrennt und
- gegebenenfalls in ihre Additionssalze mit einer pharmazeutisch annehmbaren Säure umwandelt.

7. Verfahren zur Herstellung der Verbindungen der Formel (I) nach Anspruch 1, worin R₁ = R₂, **dadurch gekennzeichnet,** daß man eine Verbindung der Formel (IX): 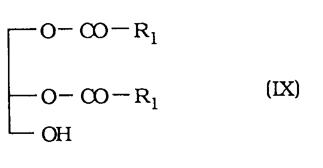 In der R₁ die bezüglich der Formel (I) angegebenen Bedeutungen besitzt,
in Form des Racemats oder eines reinen Enantiomers in Gegenwart einer Base mit dem Säurechlorid der Formel (III):
R'₃ - CO - Cl (III)
in der R'₃ eine Pyridin-3-yl-gruppe oder eine 2-Methyl-pyrazin-5-yl-gruppe darstellt, umsetzt zur Bildung der Verbindung der Formel (I/a), einem Sonderfall der Verbindungen der Formel (I): 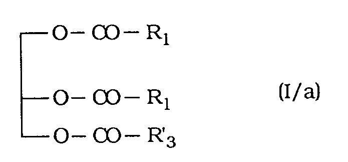 in der R₁ und R'₃ die oben angegebenen Bedeutungen besitzen,
welche Verbindung der Formel (I/a), wenn R'₃ eine 2-Methyl-pyrazin-5-yl-gruppe darstellt, einer Oxidation mit Wasserstoffperoxid in essigsaurem Medium unterworfen werden kann zur Bildung der Verbindungen der Formel (I/c), einem Sonderfall der Verbindungen der Formel (I): 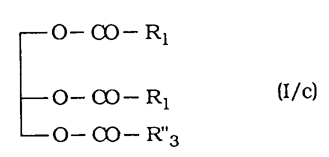 in der R₁ die bezüglich der Formel (I) angegebenen Bedeutungen besitzt und R''₃ eine 2-Methyl-1-(N-oxid)-pyrazin-5-yl-gruppe darstellt,
welche Verbindungen der Formel (I/a) oder (I/c) man:
- gegebenenfalls mit Hilfe einer klassischen Reinigungsmethode reinigt,
- gewünschtenfalls mit Hilfe einer klassischen Reinigungsmethode in ihre Isomeren auftrennt und
- gegebenenfalls in ihre Additionssalze mit einer pharmazeutisch annehmbaren Säure umwandelt.

8. Pharmazeutische Zubereitungen enthaltend als Wirkstoff mindestens eine Verbindung nach einem der Ansprüche 1 bis 5 allein oder in Kombination mit einem oder mehreren inerten, nichttoxischen, pharmazeutisch annehmbaren Trägermaterialien.

9. Pharmazeutische Zubereitungen nach Anspruch 8 zur vorbeugenden Behandlung von Arteriosklerose.
